# EUROPEAN PATENT SPECIFICATION

(11) **EP 2 301 525 B1**
(45) Date of publication and mention of the grant of the patent: **11.09.2013**
(21) Application number: 09757648.2
(22) Date of filing: 30.04.2009
(51) Int. Cl.: A61K 9/127, A61K 31/192, A61P 29/00

(54) **Topical ibuprofen formulation**
Topische Ibuprofen-Formulierung
Préparation topique d'ibuprofène

(30) Priority: 06.06.2008 ES 200801720
(43) Date of publication of application: 30.03.2011
(73) Proprietor: Laboratorios Alcala Farma S.l., 28802 Alcalá de Henares (ES)
(72) Inventor: PINILLA DE BLAS, Andrés, E-28802 Alcalá de Henares - Madrid (ES); ORTIZ VICENTE, Ana, E-28802 Alcalá de Henares - Madrid (ES); COMÍN AGUIRRE, Jaime, E-28802 Alcalá de Henares - Madrid (ES); VAYÁ IBARRA, Esther, E-28802 Alcalá de Henares - Madrid (ES); González Enseñat, Pedro, 28802 Alcalá de Henares Madrid (ES)
(74) Representative: ABG Patentes, S.L.
(86) International application number: PCT/ES2009/070131
(87) International publication number: WO 2009/147269

(56) References cited:
- EP-A2- 0 225 162
- WO-A1-88/07853
- WO-A1-93/25192
- US-A- 5 827 533
- VISHWAKARMA K K ET AL: "In vitro absorption studies of ibuprofen with cholic and deoxycholic acid conjugates", INDIAN JOURNAL OF PHARMACEUTICAL SCIENCES, vol. 60, no. 3, May 1998 (1998-05), pages 149-152, XP009165484, ISSN: 0250-474X
- A PAAVOLA: "Controlled release injectable liposomal gel of ibuprofen for epidural analgesia", INTERNATIONAL JOURNAL OF PHARMACEUTICS, vol. 199, no. 1, 10 April 2000 (2000-04-10), pages 85-93, XP55046709, ISSN: 0378-5173, DOI: 10.1016/S0378-5173(00)00376-8
- VOLONTE, M.G. ET AL.: 'Determinacion of the concentracion micelar crítica (CMC) of sales biliares by the méthe whole of tension superficial' ACTA FARM. BONAERENSE vol. 8, no. 1, 1989, pages 31 - 35, XP008140382

## Description

### Field of the Invention

The present invention relates to liposomal liquid ibuprofen formulations for topical application. The present invention particularly relates to liposomal liquid formulations which comprise bile acid salts and are suitable for their application by spraying, to processes for obtaining them and to the use of bile acids to improve the transcutaneous penetration of ibuprofen in liquid pharmaceutical compositions comprising a dispersion of liposomes.

### Background of the Invention

Oral NSAIDs have been considered as the pharmacological treatment of choice against the pain of musculoskeletal diseases of soft tissues, but their use is limited by the numerous adverse effects associated to their oral administration (especially gastrointestinal adverse effects such as digestive hemorrhage, perforated ulcer, and congestive heart failure).

Ibuprofen is a non-steroidal anti-inflammatory drug (NSAID) belonging to the group of arylpropionic derivatives and sharing its pharmacodynamic properties with the rest of the NSAIDs, which properties are mainly defined by the trilogy: analgesic activity, anti-inflammatory activity and antipyretic activity.

The established clinical use of ibuprofen admits different routes of administration, among which the oral route and the topical route by cutaneous application stand out, the latter having the advantage of being associated to a lower incidence of adverse effects.

Ibuprofen is formulated at concentrations of 5% or 10% by weight in legally authorized medicinal products for cutaneous use. When it is at 5%, it forms part of over-the-counter medicinal products, i.e., medicinal products intended for self-medication, without the need for a physician to diagnose the pathology to be treated prior to the dispensing of the medicinal product through the pharmacy. In Spain, the 5% ibuprofen has been authorized for the following therapeutic indications: local relief of mild and occasional pain and inflammation caused by: small contusions, blows, distensions, torticollis or other contractures, lower back pain and mild sprains caused as a result of a strain.

When formulated at 10%, ibuprofen can form part of over-the-counter or prescription medicinal products, depending on the pharmaceutical regulations in force in each country.

Medicinal products containing ibuprofen for their topical use in the skin have been marketed since the late 1990s. This route of administration was initially proposed as an alternative to the use of the oral NSAIDs since it had a better safety profile (local irritations being the most frequent adverse effects, in this case). Once the topical medicinal product is applied, ibuprofen penetrates slowly through the subcutaneous layers of the skin, exerting its pharmacological action locally.

However, cutaneous administration has the disadvantage that a low systemic absorption is achieved after its application (about 5-10% from the concentrations reached when the oral route is used), most of the drug is therefore wasted.

The formulations for topical application of NSAIDs are known as described in the following documents.

European patent application number EP 0364266 describes a pharmaceutical composition for restoring the function of corneal endothelial tissue comprising a non-steroidal anti-inflammatory compound (NSAID), among which ibuprofen is mentioned. It is generally mentioned that said composition can be included in a controlled-release material, among which liposomes are mentioned. Nevertheless, there is no specific mention of the combination of liposomes with ibuprofen, nor are possible compositions for said liposomes described.

Canadian patent application number CA 2500907 relates to a topical composition in the form of cream or emulsion comprising an anti-inflammatory compound as active ingredient, among which NSAIDs such as ibuprofen are mentioned. Said composition is characterized in that the oil phase of the emulsions comprises a eutectic mixture based on the combination of at least two compounds selected from camphor, L-menthol, thymol, resorcinol and phenol.

European patent application number EP 0225162 describes pharmaceutical compositions for their application by topical (although not cutaneous) route formed by liposomes or biopolymer microcapsules into which a NSAID (non-steroidal anti-inflammatory) type drug is incorporated in aqueous suspension. Said compositions are used to inhibit post-surgical adhesion formation.

International patent application number WO88/07853 describes a pharmaceutical composition, for administration by intraperitoneal route, based on liposomes formed by a non-phospholipid lipid component and a phospholipid component which incorporate an active ingredient. Specifically, liposomes formed by phosphatidylcholine and cholesterol which incorporate the sodium salt of ibuprofen as active ingredient are specified.

Patent application number WO92/09268 relates to a pharmaceutical composition based on liposomes which incorporate NSAID drugs and to their application to prevent adhesion formation between non-epithelial tissues in synovial joints. It specifically describes the formation of liposomes formed by L-alpha-distearoyl phosphatidylcholine, cholesterol and alpha-tocopherol (vitamin E) and their subsequent spray drying to form a dry powder to which the sodium salt of tolmetin is subsequently added as active ingredient.

British patent application number GB 2217596 describes pharmaceutical compositions aimed at the treatment of arthrosis, which are formed by liposomes incorporating 4-biphenylacetic acid (an NSAID known as felbinac) as active ingredient. Said compositions are administered by parenteral route. The liposomes are formed from a phospholipid, among which several phosphatidylcholines are mentioned, and can furthermore incorporate other adjuvants such as sterols (cholesterol, phytosterol, ergosterol, etc.), and antioxidants (tocopherol, propyl gallate, etc.).

European patent application number EP 1457202 relates to topical compositions comprising an NSAID as active ingredient, among which ibuprofen is mentioned, and their usefulness in alleviating pain and inflammation caused by herpes virus infection. Said compositions are in the form of gel, cream or solution but do not contain liposomes.

International patent application number WO96/29988 describes pharmaceutical compositions in the form of gel for their topical administration, formed by a polar lipid, among which lecithin or phosphatidylcholine are mentioned; a surfactant chosen from docusate sodium, docusate sodium benzoate, docusate calcium and ibuprofen; an organic solvent among which only isopropyl esters such as isopropyl myristate and isopropyl palmitate are mentioned; an inorganic base as sodium hydroxide; water; urea and an active ingredient among which ibuprofen is mentioned. The application further specifies that when ibuprofen is incorporated as active ingredient to formulations it is not necessary to add an additional surfactant.

United States patent number US 4,704,406 describes a pharmaceutical composition (which does not contain liposomes) for topical use in the form of spray containing ibuprofen as active ingredient and its use in the treatment of traumatic or rheumatic conditions.

International patent application number WO93/25192 describes pharmaceutical compositions which do not contain liposomes and mainly comprising bile acids and an NSAID active ingredient, among which ibuprofen is mentioned.

European patent application number EP 0 648 114 describes liposomes which, despite being intended for their therapeutic application, do not contain any active ingredient and which are obtained by mixing lipids capable of forming lipid bilayers and bile acids.

Further relevant prior art includes Vishwakarma KK et al.: "In vitro absorption studies of ibuprofen with cholic and deoxycholic acid conjugates", Indian Journal Of Pharmaceutical Sciences, vol.60, No.3, 1998, pages 149 - 152.

However, none of the documents known in the state of the art describes a liposomal pharmaceutical ibuprofen formulation with an improved absorption profile, which can furthermore be used for its topical administration by means of a spray and which also allows the use of active ingredient concentrations less than 3% by weight, preferably less than 2% by weight, more preferably less than 1.7% by weight, even more preferably less than 1.55% by weight.

The solution proposed by the present invention would lie in a liposomal liquid pharmaceutical composition comprising ibuprofen as active ingredient, a phospholipid, an alkanol, a bile acid salt as surfactant and water.

### Summary of the Invention

The present invention relates to a liposomal liquid pharmaceutical composition comprising ibuprofen, at least one phospholipid, at least one C₂-C₆ alkanol and water, characterized in that the liposomes comprise, in addition to at least part of the phospholipid and at least part of the ibuprofen, at least one bile acid salt and to a method for preparing it. The invention also relates to the use of the composition for obtaining a medicinal product for the local relief of pain and inflammation and to a spray for the cutaneous application of the composition.

The compositions of the present invention have an improved transcutaneous absorption profile which is shown in a higher percentage of absorbed ibuprofen with respect to the total content in the formulation and in a higher penetration rate.

This improved absorption profile makes it possible to incorporate ibuprofen in the liposomal compositions at concentrations lower than those used up until now in the formulations of the state of the art and at which authorized medicinal products with ibuprofen for cutaneous use both in the European Union and in USA currently have, in which the lowest concentration of ibuprofen is 5% by weight.

### Detailed Description of the Invention

In the present invention, liposomes are understood as essentially spherical aqueous compartments, surrounded by at least one closed lipid bilayer, which can have a diameter from 20 nm to several millimeters.

In the present invention, the term phospholipid comprises phosphoacylglycerols, i.e., compounds made up of a molecule of glycerol two of the hydroxyl groups of which are esterified by fatty acids (linear chain monocarboxylic acids with 8 to 28 carbon atoms), the third one being esterified by a phosphate group bonding the glycerol to another organic molecule by means of a phosphodiester bond). Examples of phosphoacylglycerols are phosphatidylethanolamine, phosphatidylinositol, phosphatidic acid, phosphatidylcholine and phosphatidylserine. Those complex mixtures extracted from natural products essentially comprising phosphoacylglycerols such as lecithin, for example purified soy lecithin with a phosphatidylcholine content greater than 90% and which is commercially available with the name LIPOID S 100, are also comprised within the term phospholipids.

In the present invention, the term bile acids is used to designate the group of compounds comprising cholic acid, chenodeoxycholic acid, taurocholic acid, glycocholic acid, deoxycholic acid, ursodeoxycholic acid.

In the present invention, the term ibuprofen is used to designate 2-[4-(2-methylpropyl)phenyl]propionic acid and salts thereof with pharmaceutically acceptable bases. Preferred salts include the salts of basic amino acids such as lysine or arginine. The basic amino acid forming the salt can be in the form of the L isomer or in the form of racemate. The salts of bases such as methylglucamine and the pharmaceutically acceptable salts of cations such as the salts of alkali metals, alkaline earth metals and ammonium salts are also within the scope of the invention.

In an embodiment of the present invention, the phospholipid is selected from lecithin and phosphatidylcholine and is preferably used in a ratio by weight in relation to the weight of the composition of between 2 and 10%.

In another embodiment of the present invention, the bile acid salt is selected from the alkaline salts of cholic acid and is preferably used in a ratio by weight in relation to the weight of the composition of between 0.1 and 1%.

In another embodiment of the present invention, between 2 and 10% by weight over the total of the composition of ethanol is used as alkanol.

In a preferred embodiment of the present invention, the concentration of ibuprofen or salts thereof (expressed as percentage of ibuprofen) is comprised between 1 and 3% by weight, preferably between 1 and 2% by weight, more preferably between 1.3 and 1.7% by weight, more preferably between 1.45 and 1.55% by weight. It is also preferred to use ibuprofen in the form of free acid.

These low concentrations are possible due to the fact that the liposomal liquid compositions of the present invention show, in comparison to the formulations of the state of the art, a clear improvement in the cutaneous absorption profile which is shown in a higher percentage of absorbed ibuprofen and in a fast penetration through the skin in the application site with the consequent speed of the onset of its pharmacological action.

In addition to the essential substances already described, the compositions according to the invention can incorporate from 0.1 to 1% by weight of electrolytes such as sodium chloride, 0.1 to 1% by weight of preservatives such as antimicrobial preservatives, for example phenoxyethanol and others of the class of parabens and up to 1% of flavors such as menthol.

In another aspect of the present invention the liquid pharmaceutical compositions described can, due to the fact that they have low viscosities, be incorporated in a spray for their cutaneous application by spraying.

The invention also extends to the use of the previously described compositions for the local relief of pain and inflammation.

The invention is illustrated by means of the following examples which must not be considered as limiting.

Finally, the present invention also extends to a process for preparing the previously mentioned compositions comprising the following steps:
a) preparing an aqueous solution comprising ibuprofen and the bile acid salt or salts
b) preparing a solution of the phospholipid or phospholipids in alkanol
c) mixing both solutions providing mechanical energy to the mixture
d) optionally diluting the mixture obtained in step c) in water or in an aqueous solution

### EXAMPLES

### EXAMPLE 1

### Preparation of Solution A:

14.04 g of ethanol and 18.00 g of phosphatidylcholine are incorporated in a vessel and stirred until complete dissolution.

### Preparation of Solution B:

In a vessel provided with stirring means, 0.994 g of sodium hydroxide are dissolved in 77.12 1 of demineralized water and stirred until complete dissolution. 5.400 g of ibuprofen (free acid) are added on the previous solution, stirring until being completely dissolved, and then 2.160 g of sodium cholate are added, stirring until complete dissolution. Finally, 1.080 g of sodium chloride are added and stirred until dissolving completely.

Solution B is added on Solution A and stirred for 30 minutes at a speed between 1000 and 1500 r.p.m., applying vacuum to prevent foam from being formed and maintaining the temperature constant at 25°C and obtaining a transparent yellow solution of liposomal concentrate.

### Preparation of the final product (diluted liposomes)

Once the liposomal concentrate is prepared, it is diluted in the ratio of 1:3 in demineralized water and the preparation is concluded by adding the preservative.

The resulting formulation has the composition shown in the following table:

| Components | g /100 g |
|---|---|
| Lecithin Lipoid S-100 | 5 |
| Ethanol | 3.9 |
| Sodium hydroxide | 0.276 |
| Ibuprofen (free acid) | 1.5 |
| Sodium cholate | 0.6 |
| Sodium chloride | 0.3 |
| Menthol | 0.33 |
| Phenoxyethanol | 1 |
| Water | 87.09 |

### EXAMPLE 2

### In vitro assay of permeation through pig skin.

### Material and Methods

The products subjected to the assay were the following:
- Example 1: The composition according to the invention described in Example 1
- Comparative Example 2: The commercial product Diltix^{®} which is a 5% ibuprofen solution for cutaneous spraying
- Comparative Example 3: The commercial product Nurofen^{®} gel which is a 5% ibuprofen gel for cutaneous spraying

Recently extirpated pig abdominal skin was obtained from a local slaughterhouse. The tissue was cleaned under cold running water and then dermatomed (electric dermatome ZimmerTM, OH) to a nominal thickness of 750 pm. The obtained pieces of tissues were wrapped individually in ParafilmTM and stored at a temperature of -20°C until their use.

On the day of the experiments, the necessary pieces of skin are allowed to defrost at room temperature and, then, held between the upper and lower chambers of Franz diffusion cells (PermeGear Inc, Hellertown, PA). The transport area was 1.77 cm² (1.5 cm in diameter) and the volume of the receptor chamber was 7.5 mL. The diffusion cells are thermostated using a water bath at 37°C.

The receptor solution consisted of saline phosphate buffer, pH 7.4.
40 µl of the compositions to be tested were measured and placed on the surface of the skin. This volume of formulation was sufficient to completely cover the surface. In the case of comparative composition 2, 36 mg were weighed directly and distributed uniformly on the skin.
1 mL of sample of the solution of the receptor was taken every hour for six hours and substituted with a new buffer solution. The samples were filtered (4 mm Nylon, 0.45 µm) and kept in the fridge until the analysis.

Analysis of ibuprofen: Ibuprofen was analyzed by HPLC with UV detection. The equipment was a Jasco (AS-2051, UV-2075, PU-2080). A mobile phase (55:45 Acetonitrile: citric acid, pH 2.4) was pumped (1.5 mL/min) through an Acclaim column (C18 4.6 x 150 mm). The injection volume was 50 µl. Ibuprofen was detected through its UV absorption at 264 nm. The retention time was 6 minutes. A calibration curve was obtained in the range of concentrations: 1.5-15 µg/ml. The limit of detection was 0.1 µg/mL and the limit of quantification was considered as 0.25 µg/ml. A set of standards was verified for each series of samples analyzed.

The concentration in each injected sample was calculated through the calibration curve. The amount of ibuprofen penetrated in the receptor compartment in each sampling time was estimated from the concentration, the volume of the receptor of the sample and taking into account the amount of drug eliminated from the receptor compartment in the previous samples. The percentage with respect to the total of active ingredient contained in the sample assayed was calculated from the amount of ibuprofen penetrated.

### Results

Six repetitions were performed for each composition to be assayed and the following results were obtained:

Percentage of the dose released through the dermatomed pig skin

**TABLE 1**

| %Ibuprofen released in 6 hours | | |
|---|---|---|
| | Mean | Std. Dev. |
| Ex. 1 | 3.54 | 1.59 |
| Comp. 2 | 1.1 | 0.58 |
| Comp. 3 | 0.68 | 0.64 |

The results of this *ex vivo* study summarized in Table 1 show that the percentage of dose released with the formulation according to the present invention (Example 1) is approximately three times the one released with the products of Comparative Examples 2 and 3.

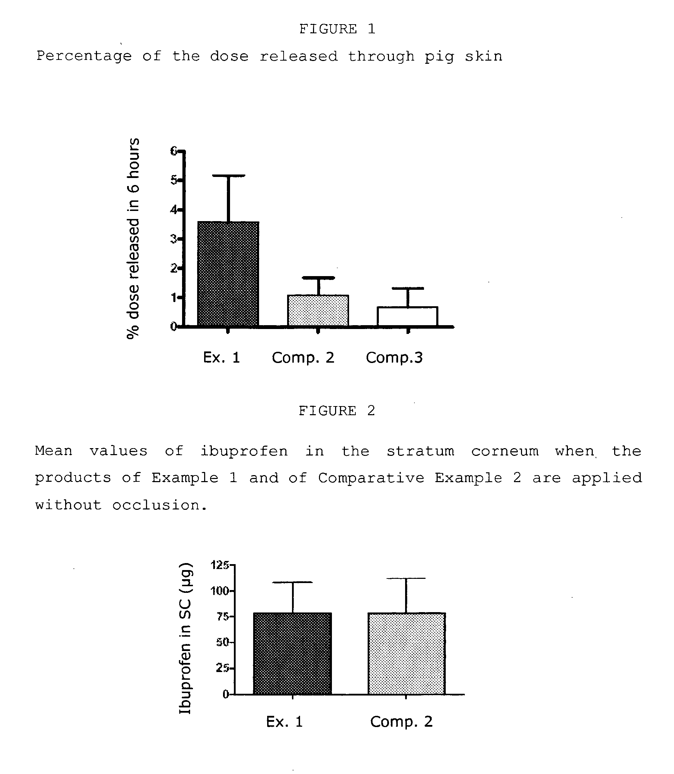

The results are graphically shown in Figure 1.

### EXAMPLE 3

### In vivo studies in humans.

### Material and Methods

The products subjected to the assay were the following:
- Example 1: The composition according to the invention described in Example 1
- Comparative Example 2: The commercial product Diltix^{®} which is a 5% ibuprofen solution for cutaneous spraying

The experiments were performed in conditions of absence of occlusion intended to simulate the actual conditions of a use application.

Two strip extraction processes were performed in three sites of the skin in the forearm for each of the participants to investigate the penetration of ibuprofen of the formulations in the stratum corneum and determine the concentration profile of the drug.

### Participants.

Four healthy volunteers (aged 24 to 44 years) without a history of dermatological disease participated in the study. The ethical approval was granted by the Salisbury Local Research Ethics Committee. The protocols of the Declaration of Helsinki were followed and the informed consent was obtained from all the volunteers.

### Thickness of the stratum corneum.

The transepidermal water loss (TEWL) through the skin was measured with a closed-chamber evaporimeter (Biox Aquaflux AF102, Biox Systems Ltd, London, United Kingdom; measurement range 0-100 g.m²h⁻¹; resolution ± 0.05 g.m²h⁻¹). The TEWL was measured three times and the mean value was taken as the baseline value.

A template (2 x 2 cm) was first applied to the surface of the skin. Then, 20 pre-weighed (Sartorius SE-2F Micro balance, precision 0.1 µg; Sartorius AG, Goettingen, Germany) strips of tape (2.5 x 2.5 cm, Scotch 845 Book tape (3M, St Paul, MN) were applied to the skin one by one and superimposing them with the template.

The strips were subsequently removed for the purpose of collecting the stratum corneum layers. After removing each strip the TEWL was measured, and the process continued until the TEWL was approximately 4 times the baseline value. The strips were weighed again to determine the amount of stratum corneum removed by each strip. Before weighing, the static electricity of each strip was eliminated using an Eltex R50 discharge bar, with an Eltex ES50 power source (Eltex Elektrostatik GmbH, Weil am Rhein, Germany).

### Ibuprofen profile in the stratum corneum:

160 µl of the compositions of Example 1 and Comparative Example 1 were applied on a surface of 2.5 x 2.5 cm for 30 min without occluding the application area. These conditions are aimed at imitating the practical application of the formulations.

Thirty minutes after the application, the excess of formulation was removed and the surface was gently cleaned using absorbent paper. The strips of strips were then sequentially removed as described above until the TEWL reached a value of approximately 4 times the baseline value or until 20 strips had been used.

Each strip was placed in a 2 mL glass vial and stirred (IKA HS 260 Basic) for 4 hours with 1 mL of CH3CN: H2O (75:25) mixture to completely extract the drug.

Ibuprofen analysis: Ibuprofen was analyzed by HPLC with UV detection. The equipment was a Jasco (AS-2051, UV-2075, PU-2080). A mobile phase (55:45 Acetonitrile: citric acid, pH 2.4) was pumped (1.5 mL/min) through an Acclaim column (C18 4.6 x 150 mm). The injection volume was 20 µl. Ibuprofen was detected through its UV absorption at 264 nm. The retention time was 6 minutes. A calibration curve was obtained in the range of concentrations: 1.5-400 µg/ml. The limit of detection was 0.1 µg/mL and the limit of quantification was considered as 0.25 µg/ml. A set of standards was verified for each series of samples analyzed.

The concentration in each injected sample was calculated through the calibration curve. The extraction of control strips with adhered stratum corneum, but without ibuprofen, was also performed and they were analyzed to verify the absence of interference peaks of substances released from the tape.

The concentration of ibuprofen in the stratum corneum, expressed as µg of ibuprofen per mg of stratum corneum, was calculated for each strip. The ibuprofen accumulated in the stratum corneum in each treated area was obtained by means of adding the ibuprofen obtained for each of the strips.

### Results

**TABLE 2**

| Ibuprofen in SC (µm) | | |
|---|---|---|
| | Mean | Std. Dev. |
| Ex. 1 | 78.21 | 30.16 |
| Comp. 2 | 78.54 | 33.56 |

**TABLE 3**

| % dose recovered in SC | | |
|---|---|---|
| | Mean | Std. Dev. |
| Ex. 1 | 3.62 | 1.40 |
| Comp. 2 | 1.09 | 0.47 |

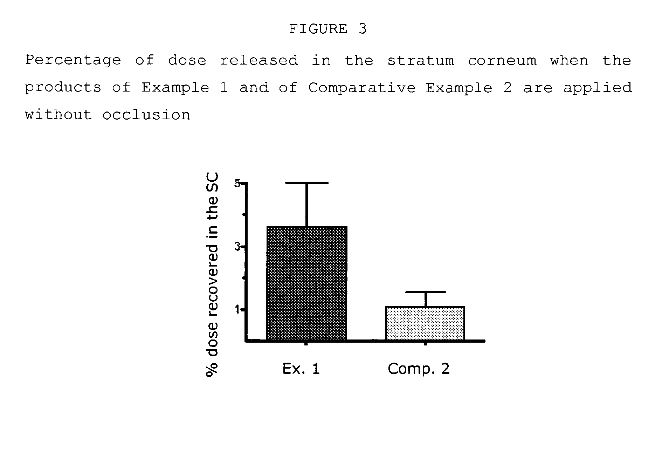

The results of Tables 2 and 3 are graphically shown in Figures 2 and 3.

As can be observed in table 3, in these studies a much higher percentage of the administered dose of ibuprofen was recovered in the stratum corneum in the composition of Example 1 according to the invention in comparison to Comparative Example 2.

This percentage was so high that the product of Example 1 could be equated to the product of Comparative Example 2 (Table 2) in the total amount of ibuprofen recovered in the stratum corneum. This data is very relevant since the product of Example 1 has 3.3 times less amount of total ibuprofen than the amount of Comparative Example 2.

To achieve this equivalence, the carrier (excipients of the formulation) of the product of Example 1 released a much higher percentage of the dose applied, the promoting effect thereof in the absorption of ibuprofen through the skin in the application site thus being demonstrated.

This fact shows that using a formula with less concentration of ibuprofen (1.5%), the total amount absorbed of the active ingredient is the same as in medicinal products marketed at 5% such as that of Comparative Example 2. This is achieved due to the fact that its absorption is more efficient and faster as a result of the novel promoting effect of the absorption of the composition according to the invention.

In this study, the authors did not visually detect any difference in the condition of the skin after the application of the different products.

## Claims

1. A liposomal liquid pharmaceutical composition for topical application comprising ibuprofen, at least one phospholipid, at least one C₂-C₆ alkanol and water, **characterized in that** the liposomes comprise, in addition to at least part of the phospholipid and at least part of the ibuprofen, at least one bile acid salt.

2. The composition according to claim 1, **characterized in that** the phospholipid is selected from lecithin and phosphatidylcholine.

3. The composition according to claim 1, **characterized in that** the bile acid salt is selected from the alkaline salts of cholic acid.

4. The composition according to any of the previous claims, **characterized in that** ibuprofen is in a ratio by weight of between 1 and 3% of the composition.

5. The composition according to claim 4, **characterized in that** ibuprofen is in a ratio by weight of between 1.3 and 1.7% of the composition.

6. The composition according to any of the previous claims, **characterized in that** the phospholipid is in a ratio by weight of between 2 and 10% and the bile acid salt is in a ratio by weight of between 0.1 and 1% of the composition.

7. The composition according to any of the previous claims, **characterized in that** between 2 and 10% by weight over the total of the composition of ethanol is used as alkanol.

8. A spray for cutaneous application comprising a pharmaceutical composition as defined in claims 1 to 7.

9. A process for obtaining a pharmaceutical composition as defined in claims 1 to 7, **characterized by** the fact that it comprises the following steps:
a) preparing an aqueous solution comprising ibuprofen and the bile acid salt or salts
b) preparing a solution of the phospholipid or phospholipids in alkanol
c) mixing both solutions providing mechanical energy to the mixture
d) optionally diluting the mixture obtained in step c) in water or in an aqueous solution

10. Use of a bile acid salt to improve the transcutaneous penetration of ibuprofen in a liposomal liquid pharmaceutical composition comprising ibuprofen, at least one phospholipid, at least one C₂-C₆ alkanol and water.

11. Use of a composition as defined in claims 1 to 7 to obtain a medicinal product for the local relief of pain and inflammation.

## Patentansprüche

1. Liposomales flüssiges Arzneimittel zur topischen Anwendung umfassend Ibuprofen, mindestens ein Phospholipid, mindestens ein C₂-C₆-Alkanol und Wasser, **dadurch gekennzeichnet, dass** die Liposomen, zusätzlich zu mindestens einem Teil der Phospholipide und mindestens einem Teil des Ibuprofens, mindestens ein Gallensäuresalz umfassen.

2. Zusammensetzung nach Anspruch 1, **dadurch gekennzeichnet, dass** das Phospholipid ausgewählt ist aus Lecithin und Phosphatidylcholine.

3. Zusammensetzung nach Anspruch 1, **dadurch gekennzeichnet, dass** das Gallensäuresalz ausgewählt ist aus alkalischen Salzen der Cholsäure.

4. Zusammensetzung nach einem der vorhergehenden Ansprüche, **dadurch gekennzeichnet, dass** Ibuprofen in einem Gewichtsverhältnis zwischen 1 und 3% der Zusammensetzung ist.

5. Zusammensetzung nach Anspruch 4, **dadurch gekennzeichnet, dass** Ibuprofen in einem Gewichtsverhältnis zwischen 1,3 und 1,7% der Zusammensetzung ist.

6. Zusammensetzung nach einem der vorhergehenden Ansprüche, **dadurch gekennzeichnet, dass** das Phospholipid in einem Gewichtsverhältnis zwischen 2 und 10% und das Gallensäuresalz in einem Gewichtsverhältnis zwischen 0,1 und 1% der Zusammensetzung ist.

7. Zusammensetzung nach einem der vorhergehenden Ansprüche, **dadurch gekennzeichnet, dass** zwischen 2 und 10 Gewichtsprozent der Gesamtmenge der Zusammensetzung des Ethanols als Alkanol verwendet wird.

8. Sprühmittel zur kutanen Verabreichung, umfassend ein Arzneimittel nach den Ansprüchen 1 bis 7.

9. Verfahren, um ein Arzneimittel nach den Ansprüchen 1 bis 7 zu erhalten, **gekennzeichnet durch** die Tatsache, dass es die folgenden Schritte umfasst:
a) Herstellen einer wässrigen Lösung, welche Ibuprofen und das Gallensäuresalz oder Gallensäuresalze umfasst
b) Herstellen einer Lösung des Phospholipids oder der Phospholipide in Alkanol
c) Mischen beider Lösungen, **durch** Bereitstellung von mechanischer Energie auf das Gemisch
d) wahlweise Verdünnen des Gemisches, das in Schritt c) erhalten wurde, in Wasser oder in einer wässrigen Flüssigkeit.

10. Verwendung eines Gallensäuresalzes zur Verbesserung der transkutanen Penetration von Ibuprofen in einem liposomalen flüssigen Arzneimittel, welches Ibuprofen, mindestens ein Phospholipid, mindestens einen C₂-C₆-Alkanol und Wasser umfasst.

11. Verwendung einer Zusammensetzung nach den Ansprüchen 1 bis 7 um ein medizinisches Produkt für die lokale Linderung von Schmerz und Entzündung zu erhalten.

## Revendications

1. Composition pharmaceutique liquide liposomique pour application topique comprenant de l'ibuprofène, au mois un phospholipide, au moins un alcanol en C₂ à C₆ et de l'eau, **caractérisée en ce que** les liposomes comprennent, en plus d'au moins une partie du phospholipide et d'au moins une partie de l'ibuprofène, au moins un sel d'acide biliaire.

2. Composition selon la revendication 1, **caractérisée en ce que** le phospholipide est choisi parmi la lécithine et la phosphatidylcholine.

3. Composition selon la revendication 1, **caractérisé en ce que** le sel d'acide biliaire est choisi parmi les sels alcalins d'acide cholique.

4. Composition selon l'une quelconque des revendications précédentes, **caractérisée en ce que** l'ibuprofène se situe dans un rapport en poids entre 1 et 3 % de la composition.

5. Composition selon la revendication 4, **caractérisée en ce que** l'ibuprofène se situe dans un rapport en poids entre 1,3 et 1,7 % de la composition.

6. Composition selon l'une quelconque des revendications précédentes, **caractérisée en ce que** le phospholipide se situe dans un rapport en poids entre 2 et 10 % et le sel d'acide biliaire se situe dans un rapport en poids entre 0,1 et 1 % de la composition.

7. Composition selon l'une quelconque des revendications précédentes, **caractérisée en ce que** entre 2 et 10 % en poids sur le total de la composition d'éthanol sont utilisés comme alcanol.

8. Spray pour une application cutanée comprenant une composition pharmaceutique telle que définie dans les revendications 1 à 7.

9. Procédé pour obtenir une composition pharmaceutique telle que définie dans les revendications 1 à 7, **caractérisé par le fait qu'**il comprend les étapes suivantes :
a) la préparation d'une solution aqueuse comprenant de l'ibuprofène et le ou les sels d'acide biliaire
b) la préparation d'une solution du ou des phospholipides dans l'alcanol
c) le mélange des deux solutions en fournissant une énergie mécanique au mélange
d) éventuellement la dilution du mélange obtenu dans l'étape c) dans de l'eau ou dans une solution aqueuse.

10. Utilisation d'un sel d'acide biliaire pour améliorer la pénétration transcutanée de l'ibuprofène dans une composition pharmaceutique liquide liposomique comprenant de l'ibuprofène, au moins un phospholipide, au moins un alcanol en C₂ à C₆ et de l'eau.

11. Utilisation d'une composition telle que définie dans les revendications 1 à 7 pour obtenir un produit médical pour le soulagement local de la douleur et de l'inflammation.
